# EUROPÄISCHE PATENTSCHRIFT

(11) **EP 1 763 363 B1**
(45) Veröffentlichungstag und Bekanntmachung des Hinweises auf die Patenterteilung: **03.06.2009**
(21) Anmeldenummer: 05758617.4
(22) Anmeldetag: 06.07.2005
(51) Int. Cl.: A61K 38/44, A23L 1/305

(54) **PHARMAZEUTISCHE ZUSAMMENSETZUNGEN, DIE DIAMINOOXIDASE ENTHALTEN**
DIAMINOOXIDASE-CONTAINING PHARMACEUTICAL COMPOSITIONS
COMPOSITIONS PHARMACEUTIQUES CONTENANT DE LA DIAMINO-OXYDASE

(30) Priorität: 07.07.2004 AT 11502004
(43) Veröffentlichungstag der Anmeldung: 21.03.2007
(60) Teilanmeldung: 07101239.7
(73) Patentinhaber: Missbichler, Albert, 1210 Wien (AT)
(72) Erfinder: MISSBICHLER, Albert, A-1210 Wien (AT); GABOR, Franz, A-3385 Gerersdorf (AT); REICHL, Herwig, A-8262 Ilz (AT)
(74) Vertreter: Sonn & Partner Patentanwälte
(86) Internationale Anmeldenummer: PCT/EP2005/053234
(87) Internationale Veröffentlichungsnummer: WO 2006/003213

(56) Entgegenhaltungen:
- EP-A- 0 132 674
- WO-A-02/066669
- FR-A- 2 101 095
- FR-A- 2 215 944
- US-A- 3 639 579
- US-A- 3 721 733
- US-A- 4 652 449
- US-A- 4 725 540
- US-A- 5 270 033
- US-A1- 2004 115 189

## Beschreibung

Die vorliegende Erfindung betrifft Zusammensetzungen zur Behandlung bzw. zur Vorbeugung von Histamin-induzierten Krankheiten und Krankheitszuständen.

Histamin (1H-Imidazol-4-Ethylamin) entsteht durch enzymatische Decarboxylierung von Histidin und ist somit ein basisches biogenes Amin mit einem Molekulargewicht von 111 Da.

Im Organismus kommt Histamin praktisch ubiquitär vor. Es wird vom Menschen selbst produziert und in den metachromatischen Granula der Mastzellen und basophilen Leukozyten in inaktiver Form gespeichert, wo es zur sofortigen Freisetzung zur Verfügung steht. Die höchsten Histamin-Konzentrationen werden in der Lunge gemessen. Nach der Freisetzung ist Histamin ein äußerst potenter Mediator einer Vielzahl von pyhsiologischen und pathophysiologischen Prozessen, oftmals auch über Wechselwirkung mit Zytokinen.

Darüber hinaus kann Histamin auch von außen in den Körper gelangen, einerseits durch Einatmen oder aber auf dem oralen Weg, beispielsweise durch die Aufnahme von histaminhältigen Lebensmitteln, wie Käse, Wein, Fischkonserven und Sauerkraut.

Die wichtigsten Funktionen und Wirkungen von Histamin im menschlichen bzw. tierischen Körper sind:
1)Dilatation der Kapillaren, Erhöhung der Kapillarpermeabilität und Blutdruckabfall.
2)Kontraktionen der glatten Muskulatur, u.a. der Bronchialmuskeln in der Lunge.
3)Induktion einer erhöhten Magensäuresekretion.
4)Erhöhung der Herzfrequenz.
5)Histamin ist der Mediator der allergischen Soforttyp-Reaktion, es ist der wichtigste Mediator bei allergischen Erkrankungen, wie Rhinitis allergica (Heuschnupfen) und Asthma bronchiale.
6)Darüber hinaus ist Histamin der klassische Auslöser einer Urticaria (Nesselausschlag) und spielt bei Medikamenten-Allergien bzw. Unverträglichkeiten eine wichtige Rolle.

Hohe Konzentrationen von freiem zirkulierendem Histamin lösen unerwünschte Wirkungen aus wie Kopfschmerzen, verlegte bzw. rinnende Nase, Atemwegsobstruktionen, Tachykardie sowie Extrasystolen, weiters Magen- und Darmbeschwerden, die zu weichem Stuhl bis Durchfällen führen können, und Hypotonie. Oft werden auch Schwellungen der Augenlider, gelegentlich auch urticarielle Exantheme beschrieben. Des Weiteren können Hautrötungen, Blutdruckabfall und Bronchospasmen auftreten. Folgende Tabelle zeigt die Symptome in Abhängigkeit von der Histaminkonzentration im Blut.

| **Histamin** **(ng/ml)** | **Körperreaktionen** |
|---|---|
| 0-1 | keine |
| 1-2 | Verstärkte Magensaftsekretion |
| 3-5 | Tachykardie, Hautirritationen |
| 6-8 | Blutdruckabfall |
| 7-12 | Bronchospasmus |
| approx.100 | Herzstillstand |

Im Organismus von Säugetieren wird Histamin von zwei Enzymen abgebaut: Diaminooxidase (DAO, EC 1.4.3.6) und Histamin-N-Methyltransferase (NMT, EC 2.1.1.8) (Mizuguchi et al. 1994). DAO katalysiert die oxidative Deaminierung von Histamin zu Imidazolazetaldehyd; NMT katalysiert die N-methylierung zu N-Methylhistamin.

Beide Abbauwege sind für den Organismus essentiell: DAO entfernt Histamin, das beispielsweise über die Nahrung in den Gastrointestinaltrakt aufgenommen wurde, NMT steuert die histaminerge Signalübertragung im Nervensystem (Kitanaka et al. 2002).

Die Hauptaufgabe der DAO ist es zu verhindern, dass über die Nahrung aufgenommenes Histamin aus dem Darm in den Blutkreislauf gelangt. Versagt dieser Schutzmechanismus, kann es im Extremfall auch zu einem anapyhlaktischen Schock kommen (Taylor 1986, Nilsson et al. 1996). DAO ist ein sekretorisches Protein und arbeitet daher extrazellulär, wohingegen N-Methyltransferase ausschließlich im Zytosol aktiv ist (Küfner et al. 2001).

Die native DAO, die neben Histamin weitere biogene Amine, wie beispielsweise Putrescin, Spermidin und Kadaverin, abbauen kann, und die beispielsweise aus Schweineniere gewonnen wird, ist ein homodimeres kupferhältiges Glycoprotein, bei welchem die Untereinheiten über Disulfidbrücken verbunden sind. DAO hat ein Molekulargewicht von etwa 182 kDa (Kluetz and Schmidt 1997, Rinaldi et al. 1982) und einen Kohlenhydratanteil von etwa 11% (Shah and Ali 1988). Das Enzym gehört zur Klasse der kupferhältigen Aminooxidasen, welche die oxidative Deaminierung von primären Aminen zu Aldehyden, Ammoniak und Wasserstoffperoxid nach dem folgenden allgemeinen Reaktionsschema katalysieren (Bachrach 1985): RCH₂NH₂ + H₂O + O₂ => RCHO + NH₃ + H₂O₂, wobei der Rest R eine Aminogruppe enthält.

Charakteristisch für die kupferhältigen Aminooxidasen ist ein Topachinon im aktiven Zentrum, das durch post-translationale Modifikation eines konservierten Tyrosin-Rests entsteht (James et al. 1990, James et al. 1992, Mu et al. 1992).

DAO ist hauptsächlich im Dünndarm, in der Leber, in den Nieren und im Blut in weißen Blutzellen zu finden. Bei Schwangeren wird DAO zusätzlich in der Plazenta gebildet. Schwangere haben etwa 500- bis 1000-mal höhere Blut-DAO-Spiegel als Nicht-Schwangere. DAO wird kontinuierlich produziert und in das Darmlumen ausgeschieden. Bei einem gesunden Menschen wird Histamin-reiche Nahrung daher bereits im Darm von Histamin weitgehend befreit. Das verbleibende Histamin wird beim Durchtritt durch die Darmschleimhaut von der dort befindlichen DAO abgebaut. Histamin wird zu Imidazolacetaldehyd und weiters - zu Imidazol-acetessigsäure zerlegt. Die Co-Faktoren der DAO sind 6-Hydroxydopa und wahrscheinlich Pyridoxalphosphat, das Vitamin B6. DAO ist ein empfindliches Enzym, das von verschiedenen Substanzen, wie anderen biogenen Aminen, Alkohol und seinem Abbauprodukt Acetaldehyd und verschiedenen Medikamenten, gehemmt werden kann. In neuronalem Gewebe konnte bisher keine DAO-Aktivität nachgewiesen werden.

Wie bereits zuvor erwähnt kann über die Nahrung aufgenommenes, körperfremdes Histamin aber auch körpereigenes Histamin aufgrund von allergischen Reaktionen eine Vielfalt von Störungen auslösen. Hinsichtlich der klinischen Wertigkeit sind dabei mindestens drei Formen einer Histamin-Intoleranz auf der Basis einer verminderten DAO-Aktivität hervorzuheben:
- Wenige Menschen haben einen angeborenen DAO-Mangel und verlieren diesen auch nicht.
- Während eines Infektes der Darmschleimhaut kann ein vorübergehender DAO-Mangel auftreten. Nach Abheilen des Infektes normalisiert sich auch die DAO-Aktivität.

US 2004/115189 beschreibt die verwendung von Histaminase zur Behandlung von Histamin induzierten Krankheiten.
- Im Rahmen der Gabe verschiedener aktivitätshemmender Substanzen kann es exogen zu einer verminderten DAO-Aktivität kommen. Dazu gehören vorrangig Alkohol und sein Abbauprodukt Acetaldehyd, gewisse aminreiche Nahrungsmittel und viele Medikamente.

In allen Fällen treten die eingangs beschriebenen Symptome mehr oder weniger massiv auf und lassen sich in den meisten Fällen nicht einfach zuordnen. Eine schnelle Abklärung der funktionellen Aktivität des Enzyms erlaubt eine rasche und einfache Therapie und die Erstellung eines entsprechenden Diätplans.

Ein häufiger Grund für das Auftreten von Histamin-Intoleranz ist die Sensibilität des Enzyms gegenüber einer Vielzahl von chemischen Substanzen. Viele davon kommen in verschiedenen Arzneimitteln vor. Die wichtigsten DAO-Inhibitoren sind Acriflavine, Diazepam, N-Methyl-N-Formylhydrazin, b-Aminopropionitrile, Dimaprit, O-Methylhydroxylamin, Agmantine, Ethanol (10%), Pargylin, Aldomet, Furosemid, Phenamil, Amilorid, Guanabenz, Phenelzin, Aminoguanidin, Guanfacin, Phenformin, Amitryptilin, Guanidin, Phenyprazin, Amodiaquin, Haloperidol, Promethiazin, Anserin, Hyamin 1622, Propranolol, Aziridinylalkylamine, Hydroxychloroquin, B1 Pyrimidin, Hydroxylamin, Quinacrin, Burimamid, Impromidin, Semicarbazid, Cadaverin, Imidazolderivate, Thiamin, Carnosin, Iproniazid, Thioridazin, Chlorothiazid, Isocarboxazid, Tranylcypramin, Chlorpromazin, Isoniazid, Trimethoprim, Cimetidin, Metiamid, Tryptamin, Clonidin, Metronidazol, Tyramin, Cyanid, Nazlinin (Alkaloid), Diamine (auch Histamin) und Nt-Methylhistamin.

In der WO 02/43745 wird die systemische Verwendung von DAO pflanzlichen Ursprungs zur Behandlung von Histamin-vermittelten Erkrankungen offenbart. Die Verabreichung von DAO oder von Enzymen allgemein, die aus Pflanzen direkt isoliert werden, ist aufgrund des häufigen Vorkommens von Allergenen in Pflanzen sehr problematisch, vor allem in Anbetracht der Tatsache, dass die in der WO 02/43745 offenbarten Hülsenfrüchte ein starkes allergenes Potential aufweisen.

Aufgabe der vorliegenden Erfindung ist es, Zusammensetzungen zur Behandlung und zur Prävention von Histamin-induzierten Krankheiten und Krankheitszuständen zur Verfügung zu stellen, die die Nebenwirkungen der am Markt befindlichen Produkte, die vorwiegend Cortison umfassen, nicht aufweisen und die Nachteile des oben angeführten Standes der Technik beseitigen. Eine weitere Aufgabe der vorliegenden Erfindung ist es die Konzentration von aktiver Diaminooxidase im Körper, insbesondere im Darmtrakt, eines Individuums zu erhöhen, um dadurch den Abbau von insbesondere exogen (z.B. durch Nahrung) zugeführtem Histamin zu unterstützen bzw. zu ermöglichen.

Daher betrifft die vorliegende Erfindung pharmazeutische Zusammensetzungen zur Behandlung von Histamin-induzierten Krankheiten, umfassend Diaminooxidase, wobei die Zusammensetzung in einer Darreichungsform zur oralen oder peroralen Verabreichung, in einer Hapensäure geschützten Darreichungsform vorliegt.

Die Diaminooxidase liegt in den erfindungsgemäßen Zusammensetzungen im Wesentlichen in dessen aktiver Form vor, d.h. dass Enzyme, die beispielsweise kein Kupfer in deren prosthetischen Gruppe aufweisen und nicht zumindest Wild-Typ Aktivität zeigen, nicht für die erfindungsgemäßen Verwendungen geeignet sind.

Die erfindungsgemäße pharmazeutische Zusammensetzung weist eine Darreichungsform auf, die eine Verabreichung ausgewählt aus der Gruppe bestehend aus oral und peroral ermöglicht. Durch die orale und perorale Verabreichung der erfindungsgemäßen Zusammensetzung ist es möglich DAO in den Magen-Darm-Trakt eines Individuums zu bringen und dort Histamin-induzierte Krankheiten durch Abbau von Histamin erfolgreich zu unterbinden bzw. zu behandeln. Der Wirkungsbereich der DAO beschränkt sich dabei vorwiegend auf den Darm-Trakt, da der hohe Säuregehalt im Magen sich negativ auf die Aktivität von DAO auswirkt. Daher ist es erforderlich bei oraler bzw. peroraler Verabreichung DAO bis zum Erreichen des Darm-Traktes vor Magensäure zu schützen.

Die pharmazeutische Zusammensetzung wird in einer Darreichungsform, ausgewählt aus der Gruppe bestehend aus magensaftresistenten Pellet, Tabletten und Kapseln zur Verfügung gestellt. Gemäß der vorliegenden Erfindung ist es möglich, DAO durch entsprechend den im Stand der Technik bekannten Aufbereitungsmethoden in pharmazeutischen Darreichungsformen zu überführen. Daher enthalten pharmazeutische Zusammensetzungen, die DAO beinhalten, auch weitere Inhaltstoffe, die verwendet werden, um einerseits das Enzym zu stabilisieren und andererseits das Enzym in die entsprechende galenische Form zu bringen. DAO kann natürlich auch mit anderen pharmazeutisch aktiven Wirkstoffen gemeinsam in einer einzigen Darreichungsform oder getrennt verabreicht werden, Sofern das Enzym nicht durch einen dieser Wirkstoffe dermaßen inhibiert wird, dass eine Aktivität des Enzyms nicht die gewünschte Wirkung entfaltet.

Ein weiterer Aspekt der vorliegenden Erfindung betrifft eine Nahrungsergänzungsmittel-Zusammensetzung bzw. ein diätetisches Lebensmittel (DLM), die/das als Magensaftresistentes Pellet, Tropfen oder Infusion adaptiert ist. Die orale Verabreichung von DAO mittels eines Nahrungsmittels, Nahrungsergänzungsmittels bzw. diätetischen Lebensmittels setzt voraus, dass dieses Enzym in jenen Bereich des Körpers gelangt, in dem es seine Wirkung entfalten soll. Da Histamin im Darm-Trakt eine bedeutende Rolle spielt, ist es notwendig, dass DAO in der Weise adaptiert bzw. vorgesehen wird, dass es unbeeinträchtigt den stark säurehaltigen Magen passieren kann. Vorzugsweise ist dabei die DAO als magensaftresistentes Pellet aufgearbeitet.

Da DAO bei einem pH-Wert von unter 3 irreversibel geschädigt wird (im Magen liegt der pH-Wert zwischen pH 2 und 4) ist es notwendig, um die DAO durch den Magen in den Darmtrakt zu transportieren, dass die DAO in einer entsprechenden Darreichungsform (pharmazeutische bzw. Nahrungsergänzungsmittel-Zusammensetzung bzw. diätetisches Lebensmittel) vorgesehen ist. Erfindungsgemäß haben sich Kapseln (z.B. Gelatine-Kapseln) und insbesondere magensaftresistente Pellets als besonders vorteilhaft erwiesen. Es ist von Vorteil wenn die Wirkung der DAO spätestens 15, vorzugsweise spätestens 20, insbesondere spätestens 30, Minuten nach Verabreichung eintritt.

Erfindungsgemäß wird als "magensaftresistent" die Eigenschaft des Pellets bezeichnet, die ein darin enthaltenen Wirkstoff (z.B. DAO) bei Einwirkung eines Magensafts bzw. einer Lösung die vergleichbare Eigenschaften wie Magensaft aufweist (z.B. Säure) für einen bestimmten Zeitraum von mindestens 10, vorzugsweise mindestens 20, noch mehr bevorzugt mindestens 30, insbesondere mindestens 60, Minuten derart zu schützen vermag, dass der Wirkstoff ein Aktivitätsverlust von maximal 50%, vorzugsweise von maximal 40%, noch mehr bevorzugt von maximal 30%, am meisten bevorzugt maximal 20%, insbesondere maximal 10%, erfährt.

Vorzugsweise setzt das erfindungsgemäße Pellet nach 20 Minuten, insbesondere nach 30 Minuten, mindestens 60%, insbesondere mindestens 80%, der DAO-Aktivität, die zur Formulierung der Pellets eingesetzt wurde, im Darm frei.

Magensaftresistente Pellets sind Pellets, die von einem magensaftresistenten Überzug umhüllt sind, die sich bei einem pH-Wert auflösen wie der im Darmtrakt vorzufinden ist. D.h. derartige Überzüge lösen sich somit vorzugsweise bei einem pH-Wert von mindestens 4 und maximal 10 auf. Eudragit, beispielsweise, ein magensaftresistenter Überzug basierend auf anionischen Polymeren von Methacrylsäure und Methacrylaten, enthält -COOH als funktionelle Gruppe und löst sich im Bereich von pH 5,5 bis pH 7 auf. Alternativ zu Eudragit können Schellack oder acetylierte Stärke (z.B. Amprac 01) herangezogen werden. Da die im Stand der Technik bekannten magensaftresistenten Überzüge verschiedene Eigenschaften aufweisen (z.B. pH-Wert bei der sich der Überzug auflöst, Auflösgeschwinidgkeit) können die Materialien der Überzüge auch kombiniert werden. Schellack, beispielsweise, zeigt eine gute Säureresistenz, löst sich allerdings sehr langsam im Darmtrakt auf. Amprac 01 hingegen löst sich rasch im Darmmilieu auf, zeigt jedoch keine ausreichende Säuresesistenz. Um die Nachteile eines Materials zu kompensieren können die beiden oben genannten Materialien beispielsweise in einem Gewichtsverhältnis von 60-95/40-5, vorzugsweise von 70-90/30-10, Schellack/Amprac 01 gemischt werden. Ein weiterer Parameter der die Freisetzungsgeschwindigkeit des Wirkstoffs beeinflusst ist die Schichtdicke des magensaftresistenten Pellets. Die Schichtdicke, ausgedrückt als Massenverhältnis, ist dabei vorzugsweise 5 bis 30%, noch mehr bevorzugt 10 bis 20% der Gesamtmasse des Endprodukts. Die Pellets weisen vorzugsweise einen durchschnittlichen Durchmesser von 0,5 bis 5 mm, insbesondere von 0,7 bis 2 mm, auf. Eine derartige Größe hat den Vorteil, dass die Pellets den Magen schnell passieren können.

Die Herstellung der erfindungsgemäßen Pellets, die sowohl in der erfindungsgemäßen pharmazeutischen Zusammensetzung als auch in der erfindungsgemäßen Nahrungsergänzungsmittel-Zusammensetzung bzw. diätetisches Lebensmittel Anwendung finden, erfolgt vorzugsweise mit einem Extruder, der eine thermische Stabilität der Inhaltsstoffe der Zusammensetzung, insbesondere des Wirkstoffs DAO, von bis zu 60°C erforderlich macht (Stricker Arzneiformenentwicklung, Spinger Verlag 2003). Die Pellets können neben einem magensaftresistenten Überzug und der DAO weitere pharmazeutische Zusatzstoffe umfassen. Beispielsweise dient microkristalline Zellulose (z.B. Avicel) als Füllstoff und Quellstoff. Zellulose ist unlöslich in Wasser, hat in dieser Form sowohl kristalline als auch amorphe Anteile. Diese Kombination bewirkt eine plastische Verformbarkeit, das heißt, dass bei genügend hoher Kraft eine irreversible Formveränderung eintritt. Dies ist eine wesentliche Voraussetzung für das Pelletieren im Extruder und Spheronizer. Bei der feuchten Granulierung nimmt mikrokristalline Zellulose große Wassermengen auf und wird dadurch auch ohne Bindemittelzusatz zu einer gut komprimierbaren, zusammenhaltenden Masse. Die Menge an mikrokristalliner Zellulose in einem Pellet kann erfindungsgemäß zwischen 5 und 70%, vorzugsweise zwischen 10 und 60%, noch mehr bevorzugt zwischen 15 und 50%, betragen. Als Binde- und Füllmittel kann Saccharose verwendet werden. Saccharose erhöht die Löslichkeit der Matrix und unterstützt damit die rasche Freisetzung des Enzyms. Saccharose kann erfindungsgemäß zu 1 bis 40%, vorzugsweise 5 bis 35%, noch mehr bevorzugt 10 bis 30%, einem Pellet zugesetzt werden. Hydroxypropylcellulose (zugesetzt in einer Menge von vorzugsweise 0,5 bis 10%) kann ebenfalls Bindemittel zugesetzt werden und diesnt zur Verhinderung von Feinstaub. Ferner erhöht Hydroxypropylcellulose die Festigkeit der Pellets und trägt somit wiederum zur Verbesserung der Ausbeute bei. Maisstärke kann als Füll- und Sprengmittel dem erfindungsgemäßen Pellet zugesetzt werden (in einer bevorzugten Menge von 1 bis 30%). Als wasserunlösliche Substanz kann Stärke viel Wasser aufnehmen und ist daher ein ideales Sprengmittel. Crosscarmellose (Na-CMC; Acdisol) ist ein reines Sprengmittel, das vorzugsweise in einer Menge zwischen 1% und 5% eingesetzt werden kann. Ein zu hoher Anteil an Acdisol führt zu frühem Zerfall der Pellets bereits beim Ausrunden und ist daher kontrakproduktiv. Crosspovidon, ein quervernetztes PVP, ist ebenfalls wasserunlöslich und dient ebenfalls als Sprengmittel. Aufgrund seiner Polymereigenschaften unterstützt es die bessere Ausrundung bei der Herstellung von Pellets (kann vorzugsweise in einer Menge von 0,5 bis 10% zugesetzt werden). Povidon ist ein wasserlöslicher Zusatzstoff und dient als Bindmittel. Die Kombination dieser verschiedenen Füll-, Spreng- und Bindemittel führt zu einer molekulardispersen Verteilung der DAO im Pellet und sichert eine rasche Bioverfügbarkeit.

Zwischen dem magenresistenten Überzug und dem Pellet mit dem Wirkstoff kann eine Isolierschicht aus Glycerin und/oder Talkum vorgesehen sein. Glycerin dient als Feuchthaltemittel, um eine Dehydrierung und damit Inaktivierung des Enzyms zu verhindern.

Alternativ zu Pellets kann die DAO auch in Kapseln durch den Magen in den Darmtrakt transportiert werden. Geeignete Kapseln sind dabei z.B. Gelatine-Kapseln oder Stärkekapseln. Die Kapseln können auch die erfindungsgemäßen Pellets beinhalten.

Vorzugsweise ist die Diaminooxidase, die in den erfindungsgemäßen Zusammensetzungen Verwendung findet, nicht-pflanzlichen Urpsrungs.

Die Verwendung von DAO nicht pflanzlichen Ursprungs in pharmazeutischen Zusammensetzungen sowie Nahrungsergänzungsmitteln und diätetischen Lebensmitteln hat den Vorteil, dass in Pflanzen vorkommende Allergene die Verabreichung von DAO nicht negativ beeinflusst, da Allergene die körpereigene Histaminausschüttung wesentlich fördern. Es ist bekannt, dass vor allem Pflanzenstoffe für Histamin-induzierte Krankheiten verantwortlich sind. Die vollständige Entfernung allergieauslösender Inhaltstoffe aus einer DAO-Präparation pflanzlichen Ursprungs ist nur mit hohem präparativen Aufwand möglich, hingegen ist die erfindungsgemäße DAO, welche einen nicht pflanzlichen Ursprung aufweist gänzlich frei von derartigen pflanzlichen Allergenen.

Gemäß der vorliegenden Erfindung werden unter "nicht pflanzlichen Ursprung" alle DAO umfasst, die nicht aus Pflanzen sondern aus tierischen Organismen oder sonstigen nicht pflanzlichen Organismen gewonnen werden. Des Weiteren fallen erfindungsgemäß unter diese Definition alle DAO, die rekombinant in Zellkulturen (tierischen, bakteriellen, Hefen und dgl.) bzw. in nicht pflanzlichen Organismen jeglicher Art hergestellt werden, wobei die DNA für die rekombinant hergestellte DAO von pflanzlichen und/oder tierischen Organismen mittels im Stand der Technik bekannten Methoden isoliert und in Expressionssysteme kloniert und exprimiert wird.

Vorzugsweise umfassen alle in der vorliegenden Erfindung offenbarten Zusammensetzungen Diaminooxidase tierischen Ursprungs. Durch die Verwendung von tierischem DAO ist es möglich, dem menschlichen bzw. tierischen Körper ein Enzym zuzuführen, das dem von diesen Individuen selbst hergestellten Enzym bezüglich Glykosilierung, Aktivität und Spezifität der vom diesen Individuum selbst produzierten DAO sehr nahe kommt. Weiters ist es möglich pflanzliche Allergene zur Gänze von der Herstellung von DAO auszuschließen.

Vorzugsweise wird die Diaminooxidase aus Schweinenieren gewonnen. Schweinenieren zeichnen sich vor allem durch ihren hohen Gehalt an DAO aus. Aus Schweineniere kann das Enzym in einfacher Weise mit den im Stand der Technik bekannten Methoden isoliert werden.

Gemäß einer weiteren bevorzugten Ausführungsform umfassen die erfindungsgemäßen Zusammensetzungen Diaminooxidase rekombinanten Ursprungs. Durch die rekombinante Herstellung von DAO ist es möglich, große Mengen an Enzym herzustellen und dieses Enzym in einer hohen Ausbeute zu reinigen.

Vorzugsweise wird die rekombinante Diaminooxidase in prokaryotischen, vorzugsweise in bakteriellen, oder eukaryotischen, vorzugsweise in tierischen oder Hefe-, Zellkulturen exprimiert und aus den oben genannten Expressionssystemen isoliert. Die Aufreinigung mittels dieser Expressionssysteme hergestellten DAO, die entweder in den Zellen exprimiert oder aus den Zellen während der Expression ausgeschleust wird, erfolgt durch die im Stand der Technik bekannten Methoden. Dabei ist es auch möglich die DAO mit einer Peptid- (z.B. His-Tag), Polypeptid- oder Proteinsequenz (z.B. GST-Tag) zu versehen um die Aufreinigung zu vereinfachen. Die rekombinante DAO kann weiters durch gentechnische Verfahren derart modifiziert sein, dass die Enzymaktivität dieser DAO die Enzymaktivität der Wild-Typ DAO übertrifft.

Ein weiterer Aspekt der vorliegenden Erfindung betrifft die Verwendung der erfindungsgemäßen Diaminooxidase zur Herstellung eines Arzneimittels zur Behandlung von Histamin-induzierten Krankheitsbildern. DAO ist bekannterweise für den Abbau von Histamin im menschlichen und tierischen Körper verantwortlich. Da Histamin-induzierte Krankheiten durch einen Überschuss von Histamin, das aufgrund eines Mangels an Diaminooxidase, durch Inhibition von DAO oder durch einen Histaminüberschuss, der in der Regel durch Nahrungsmittel, aber auch durch weitere extrinsische Faktoren, wie z.B. Kontakt mit Allergenen, hervorgerufen werden, eignet sich die Verabreichung von erfindungsgemäßer DAO zur Behandlung dieser Krankheiten bzw. Krankheitsbilder.

Ein weiterer Aspekt der vorliegenden Erfindung betrifft die Verwendung der erfindungsgemäßen Diaminooxidase zur Herstellung eines Arzneimittels zur Behandlung von Urticaria, insbesondere von chronischer und akuter Uritkaria. Bei Urticaria kommt es bei Freisetzung von Histamin zu einer Erweiterung von Venolen und zur überhöhten Durchlässigkeit der Kapillaren mit daraus resultierendem Ödem. Durch die Verabreichung von DAO an den betroffenen Hautzonen ist es möglich, das Histamin an betroffenen Stellen abzubauen und dadurch den Juckreiz der Urticaria zu unterbinden.

Ein weiterer Aspekt der vorliegenden Erfindung betrifft die Verwendung von erfindungsgemäßer Diaminooxdiase zur Herstellung eines Arzneimittels zur Behandlung von Kontaktallergie. Kontaktallergien werden durch Substanzen (Allergene), die durch Eindringen in die Haut allergische Reaktionen auslösen, hervorgerufen. Um die durch diesen Kontakt ausgeschütteten Histamine abzubauen und somit die Histamin-induzierenden Krankheitserscheinungen zu unterbinden bzw. zu lindern, wird DAO verabreicht.

Gemäß einem weiteren Aspekt der vorliegenden Erfindung wird eine erfindungsgemäße Diaminooxidase zur Herstellung eines Arzneimittels zur Behandlung von atopischer Dermatitis verwendet. Atopische Dermatitis, auch bekannt unter dem Namen Neurodermitis, ist eine häufige Hauterkrankung mit starkem Juckreiz zumeist bei Kindern und jugendlichen Erwachsenen. Ursache dieses Juckreizes ist die übermäßige Ausschüttung von Histaminen an den betreffenden Hautstellen. Auch in diesem Fall kann DAO zur Reduktion der Histamine in diesen Bereichen beitragen und somit die Symptome von atopischer Dermatitis lindern bzw. verhindern.

Gemäß einem weiteren Aspekt betrifft die vorliegende Erfindung die Verwendung der erfindungsgemäßen Diaminooxidase zur Herstellung eines Arzneimittels zur Behandlung von Scombrotoxismus. Scombrotoxismuns ist eine Histaminvergiftung nach Genuss von Makrelenarten, z.B. von Thunfisch. Bei Unterbrechung der Kühlkette bzw. bei Verzögerung der Zubereitung bilden sich bei Scombriden sogenannte Scombrotoxine, die zu einer Histaminanreicherung führen. Folge dieser Histaminvergiftung sind unter anderem Fieber, Übelkeit, Erbrechen, Bauchschmerzen und Urticaria. DAO kann in diesem Fall als Detoxikationsmittel verwendet werden.

Ein weiterer Aspekt der vorliegenden Erfindung betrifft die Verwendung der erfindungsgemäßen Diaminooxidase zur Herstellung eines Arzneimittels oder Nahrungsergänzungsmittels bzw. diätetischen Lebensmittels zur Entfernung bzw. Reduktion von Histamin aus dem Gastrointestinaltrakt.

Die Reduktion von Histamin aus dem Darmtrakt ist von besonderer Bedeutung bei einer erhöhten Zufuhr von Histamin (exogene Zufuhr, z.B. durch Nahrungsmittel) in den Körper eines Individuums oder wenn beispielsweise die Aktivität der DAO im Darmtrakt teilweise oder zur Gänze inhibiert bzw. nicht vorhanden ist. Das erfindungsgemäße Arzneimittel bzw. Nahrungsergänzungsmittel bzw. diätetische Lebensmittel dient somit zur Zufuhr von enzymatisch aktiver DAO, die zum Abbau von Histamin im Darmtrakt beiträgt.

Die Erfindung wird weiters durch folgende Beispiele erläutert.

### BEISPIELE:

### Beispiel 1

DAO wurde in einem Pellet auf Zellulose-Basis aktiv stabilisiert und das Pellet wurde mit einer magensaftresistenten Schicht überzogen. Im Dissolutionstest konnte gezeigt werden, dass mehr als 70% der Aktivität innerhalb der ersten Stunde in die Umgebung freigesetzt wird.

### Beispiel 2 (nicht Teil der Erfindung)

Weiters wurde das Enzym in einem Hydrogel stabilisiert. Lagerung bei Raumtemperatur und 37°C zeigten keinen Abfall der Aktivität innerhalb von 4 Monaten. Auftretende Bläschen und Juckreiz an der Haut nach Stimulation mit Histamin verschwanden wenige Minuten nach Applikation des Hydrogels.

### Beispiel 3 (nicht Teil der Erfindung)

Die in Hydrogel stabilisierte DAO wurde insgesamt an 13 Personen getestet. Anhand eines Fragebogens wurde unterschieden zwischen Belastung durch Kontakallergie (n=5), Neurodermitis/Dermatosen (n=6) und Insektenstichen (n=2). Bei allen Kontaktallergikern und Neurodermitis-Patienten wurde eine positive Wirkung attestiert, bei Insektenstichen konnten 50% der Probanden von einer positiven Wirkung berichten.

| ***Belastung*** | ***Positive Wirkung*/*Anzahl Probanden*** |
|---|---|
| Neurodermitis/Dermatose | 6/6 |
| Kontaktallergie | 5/5 |
| Insektenstich | 1/2 |

Die Wirkung des Hydrogels trat innerhalb der ersten 10 Minuten ein, wobei die Wirkung durchschnittlich länger als eine Stunde anhielt.

| | ***0-10 min*** | ***10-30 min*** | ***30-60 min*** | ***> 1h*** | **> *3h*** |
|---|---|---|---|---|---|
| Wirkungsbeginn | 11 | 1 | | | |
| Wirkungsdauer | | 3 | 1 | 2 | 6 |

Kein Proband meldete unangenehme Effekte bei wiederholter Anwendung.

Zwei Neurodermitis-Patienten gaben an, dass das erfindungsgemäße Hydrogel besser als eine aktuell eingesetzte Cortison-Salbe wirkt.

### Beispiel 4

Magensaftresistente Pellets wurden mit 3% DAO, welche eine Ausgangsaktivität von 80000 E/ml aufgewiesen hat, 40% mikrokristalliner Zellulose, 20% Saccharose, 22% sonstigen Binde-, Füll- und Sprengmittel und mit 15% magensaftresistenten Überzug hergestellt. Die Freisetzung von DAO aus den Pellets wurde über eine Zeit von 180 Minuten mittels eines Aktivitätsassays beobachtet. Dabei wurde in der Lösung, in dem die Pellets aufgelöst wurden, bereits nach 10 Minuten eine DAO-Aktivität von 40%, nach 30 Minuten von 70%, nach 60 Minuten von 80% und nach 180 Minuten von 100% von der ursprünglich eingesetzten DAO-Menge festgestellt. Die Aktivitätsmessung der DAO erfolgte wie in der AT 411688 beschrieben, wobei aber zur Messung der Enzymaktivität durchwegs auch andere bekannte Verfahren verwendet werden können.

### LITERATUR:

Bachrach U., in: B. Mondovi (Ed) (1985), Structure and Functions of Amine Oxidase, CRC Press, Boca Raton, pp 5-20x Bartholomew M.J. et al (1990), Cancer 66:1539-1543
James S.M., Palcic M.M., Scaman C.H., Smith A.J., Brown D.E., Dooley D.M., Mure M., Klinman J.P. (1992), Biochemistry 31:12147-12154
James S.M., Mu D., Wemmer D., Smith A.J., Kaur S., Maltby D., Burlingame A.L., Klinman J.P. (1990), Science 248:981-987
Keskinege A., Elgun S., Yilmaz E. (2001), Biochim Biophys Acta 25:76
Kitanaka J., Kitanaka N., Tsujimura T., Terada N., Takemura M. (2002), European Journal of Pharmacology 437:179-185
Kluetz M.D., Schmidt P.G. (1977), Biochem. Biophys. Res. Comm. 76:40-45
Küfner M.A., Ulrich P., Raithel M., Schwelberger H.G. (2001), Inflamm. res. 50, Supplement 2, 96-97
Kusche J., Menningen R., Leisten L., Krakamp B. (1988), Adv. Exp. Med. Biol. 250:745-52
Kusche J., Bieganski T., Hesterberg R., Stahlknecht C.D., Feussner K.D., Stahlenberg I., Lorenz W. (1980), Agents Actions 10:110-3
Mennigen R, Bieganski T, Elbers A, Kusche J (1989), J Chromatogr B Biomed Sci Appl 27:221
Mizuguchi H., Imamura I., Takemura M., Fukui H. (1994), J. Biochem 116:631-635
Mu D., James S. M., Smith A.J., Brown D.E., Dooley D.M., Klinman J.P. (1992), J. Biol. Chem. 267:7979-7982
Nilsson B.O., Kockum I., Rosengren E. (1996), Inflammation Res. 45:513-518
Rinaldi A., Vecchini P., Floris G. (1982), Prep. Biochem. 12:11-28
Shah M. A., Ali R. (1988), Biochem. J. 253:103-107
Taylor S.L. (1986), Crit. Rev. Toxicol. 17:91-128

## Patentansprüche

1. Pharmazeutische Zusammensetzung zur Behandlung von Histamin-induzierten Krankheiten, umfassend Diaminooxidase, **dadurch gekennzeichnet, dass** die Zusammensetzung in einer Magensäure geschützten Darreichungsform zur oralen oder peroralen Verabreichung vorliegt.

2. Zusammensetzung nach Anspruch 1, **dadurch gekennzeichnet, dass** die Zusammensetzung als Magensaft-resistentes Pellet oder Kapsel vorliegt.

3. Zusammensetzung nach Anspruch 1 oder 2, **dadurch gekennzeichnet, dass** die Diaminooxidase nicht-pflanzlichen Ursprungs ist.

4. Zusammensetzung nach Anspruch 3, **dadurch gekennzeichnet, dass** die Zusammensetzung Diaminooxidase tierischen Ursprungs umfasst.

5. Zusammensetzung nach Anspruch 4, **dadurch gekennzeichnet, dass** die Diaminooxidase aus Schweineniere stammt.

6. Zusammensetzung nach einem der Ansprüche 1 bis 4, **dadurch gekennzeichnet, dass** die Zusammensetzung Diaminooxidase rekombinanten Ursprungs umfasst.

7. Zusammensetzung nach Anspruch 6, **dadurch gekennzeichnet, dass** die Zusammensetzung rekombinante Diaminooxidase gewonnen aus prokaryotischen, vorzugsweise aus bakteriellen, oder aus eukaryotischen, vorzugsweise aus tierischen oder Hefe-, Zellkulturen umfasst.

8. Verwendung einer pharmazeutischen Zusammensetzung nach einem der Ansprüche 1 bis 7 als Nahrungsergänzungsmittel oder diätetisches Lebensmittel.

9. Verwendung von Diaminooxidase zur Herstellung eines Arzneimittels zur Behandlung von Histamin-induzierten Krankheitsbildern, **dadurch gekennzeichnet, dass** das Arzneimittel in einer Magensäure geschützten Darreichungsform zur oralen oder peroralen Verabreichung vorliegt.

10. Verwendung von Diaminooxidase zur Herstellung eines Arzneimittels zur Behandlung von Urticaria, insbesondere von chronischer und akuter Urticaria, **dadurch gekennzeichnet, dass** das Arzneimittel in einer Magensäure geschützten Darreichungsform zur oralen oder peroralen Verabreichung vorliegt.

11. Verwendung von Diaminooxidase zur Herstellung eines Arzneimittels zur Behandlung von atopischer Dermatitis, **dadurch gekennzeichnet, dass** das Arzneimittel in einer Magensäure geschützten Darreichungsform zur oralen oder peroralen Verabreichung vorliegt.

12. Verwendung von Diaminooxidase zur Herstellung eines Arzneimittels zur Behandlung von Scombrotoxismus, **dadurch gekennzeichnet, dass** das Arzneimittel in einer Magensäure geschützten Darreichungsform zur oralen oder peroralen Verabreichung vorliegt.

13. Verwendung von Diaminooxidase zur Herstellung eines Arzneimittels zur Entfernung von Histamin aus dem Gastrointestinaltrakt, **dadurch gekennzeichnet, dass** das Arzneimittel in einer Magensäure geschützten Darreichungsform zur oralen oder peroralen Verabreichung vorliegt.

14. Verwendung nach einem der Ansprüche 9 bis 13, **dadurch gekennzeichnet, dass** das Arzneimittel als Magensaft-resistentes Pellet oder Kapsel vorliegt.

## Claims

1. A pharmaceutical composition for the treatment of histamine-induced diseases, comprising diaminooxidase, **characterized in that** the composition is provided in a gastric-acid-protected dosage form for oral or peroral administration.

2. The composition according to claim 1, **characterized in that** it is provided in the form of a gastric-acid-resistant pellet or caspule.

3. The composition according to claim 1 or 2, **characterized in that** the diaminooxidase is of non-plant origin.

4. The composition according to claim 3, **characterized in that** it comprises diaminooxidase of animal origin.

5. The composition according to claim 4, **characterized in that** the diaminooxidase is derived from porcine kidneys.

6. The composition according to any one of claims 1 to 4, **characterized in that** it comprises diaminooxidase of recombinant origin.

7. The composition according to claim 6, **characterized in that** it comprises recombinant diaminooxidase recovered from prokaryotic, preferably bacterial, or from eukaryotic, preferably animal or yeast, cell cultures.

8. Use of a pharmaceutical composition according to any one of claims 1 to 7 as a food supplement or a dietary foodstuff.

9. Use of diaminooxidase for preparation of a medicament for the treatment of histamine-induced clinical pictures, **characterized in that** the medicament is present in a gastric-acid-protected dosage form for oral or peroral administration.

10. Use of diaminooxidase for preparation of a medicament for the treatment of urticaria, in particular of chronic and acute urticaria, **characterized in that** the medicament is present in a gastric-acid-protected dosage form for oral or peroral administration.

11. Use of diaminooxidase for preparation of a medicament for the treatment of atopic dermatitis, **characterized in that** the medicament is present in a gastric-acid-protected dosage form for oral or peroral administration.

12. Use of diaminooxidase for preparation of a medicament for the treatment of scombrotoxism, **characterized in that** the medicament is present in a gastric-acid-protected dosage form for oral or peroral administration.

13. Use of diaminooxidase for preparation of a medicament for removing histamine from the gastro-intestinal tract, **characterized in that** the medicament is present in a gastric-acid-protected dosage form for oral or peroral administration.

14. Use according to any one of claims 9 to 13, **characterized in that** the medicament is present as a gastric-acid-resistant pellet or capsule.

## Revendications

1. Composition pharmaceutique pour le traitement des maladies induites par l'histamine comprenant de la diaminooxydase, **caractérisée en ce que** la composition est présente dans une forme d'administration protégée contre l'acide gastrique pour l'administration orale ou perorale.

2. Composition selon la revendication 1 **caractérisée en ce que** la composition est présente sous forme d'un pellet résistant au suc gastrique ou d'une capsule résistante au suc gastrique.

3. Composition selon la revendication 1 ou 2 **caractérisée en ce que** la diaminooxydase n'est pas d'origine végétale.

4. Composition selon la revendication 3 **caractérisée en ce que** la composition comprend une diaminooxydase d'origine animale.

5. Composition selon la revendication 4 **caractérisée en ce que** la diaminooxydase est issue de reins de porc.

6. Composition selon l'une des revendications 1 à 4 **caractérisée en ce que** la composition comprend une diaminooxydase d'origine recombinante.

7. Composition selon la revendication 6 **caractérisée en ce que** la composition comprend une diaminooxydase recombinante obtenue à partir de cultures cellulaires procaryotes, de préférence de cultures cellulaires bactériennes ou de cultures cellulaires eucaryotes, de préférence de cultures cellulaires animales ou de cultures cellulaires de levure.

8. Utilisation d'une composition pharmaceutique selon l'une des revendications 1 à 7 comme complément alimentaire ou comme aliment diététique.

9. Utilisation d'une diaminooxydase pour la production d'un médicament pour le traitement des tableaux morbides induits par l'histamine **caractérisée en ce que** le médicament est présent dans une forme d'administration protégée contre l'acide gastrique pour l'administration orale ou perorale.

10. Utilisation d'une diaminooxydase pour la production d'un médicament pour le traitement de l'urticaire, en particulier l'urticaire chronique et aigu, **caractérisée en ce que** le médicament est présent dans une forme d'administration protégée contre l'acide gastrique pour l'administration orale ou perorale.

11. Utilisation d'une diaminooxydase pour la production d'un médicament pour le traitement de la dermatite atopique **caractérisée en ce que** le médicament est présent dans une forme d'administration protégée contre l'acide gastrique pour l'administration orale ou perorale.

12. Utilisation d'une diaminooxydase pour la production d'un médicament pour le traitement de la scombrotoxicité **caractérisée en ce que** le médicament est présent dans une forme d'administration protégée contre l'acide gastrique pour l'administration orale ou perorale.

13. Utilisation d'une diaminooxydase pour la production d'un médicament pour retirer l'histamine des voies gastro-intestinales **caractérisée en ce que** le médicament est présent dans une forme d'administration protégée contre l'acide gastrique pour l'administration orale ou perorale.

14. Utilisation selon l'une des revendications 9 à 13 **caractérisée en ce que** le médicament est présent sous forme d'un pellet résistant au suc gastrique ou d'une capsule résistante au suc gastrique.
